(19) Europäisches Patentamt
European Patent Office
Office européen des brevets

(11) Veröffentlichungsnummer: **0 115 585**
**B1**

(12) # EUROPÄISCHE PATENTSCHRIFT

(45) Veröffentlichungstag der Patentschrift: **04.06.86**

(51) Int. Cl.$^4$: **B 01 D 19/04**, C 07 C 127/15

(21) Anmeldenummer: **83111982.1**

(22) Anmeldetag: **29.11.83**

(54) **Verfahren zur Herstellung von Entschäumern und nach diesem Verfahren produzierte Entschäumer.**

(30) Priorität: **08.12.82 DE 3245482**

(43) Veröffentlichungstag der Anmeldung:
**15.08.84 Patentblatt 84/33**

(45) Bekanntmachung des Hinweises auf die Patenterteilung:
**04.06.86 Patentblatt 86/23**

(84) Benannte Vertragsstaaten:
**AT BE CH DE FR GB IT LI LU NL SE**

(56) Entgegenhaltungen:
**DE - A - 2 043 088**
**DE - A - 2 558 040**
**DE - C - 1 102 136**
**US - A - 4 261 844**

(73) Patentinhaber: **Byk-Chemie GmbH, Abelstrasse 14, D-4230 Wesel (DE)**

(72) Erfinder: **Haubennestel, Karlheinz, Chem. Ing. grad., Hermann Hesse Strasse 30, D-4230 Wesel 1 (DE)**
Erfinder: **Bubat, Alfred, Gotenstrasse 19, D-4230 Wesel-Bislich (DE)**
Erfinder: **Betcke, geb. Breil, Daniela, Rathausstrasse 5, D-4236 Hamminkeln (DE)**
Erfinder: **Spratte, Werner, Dr., Dipl.-Chem., Flürener Weg 82, D-4230 Wesel-Flüren (DE)**

(74) Vertreter: **Frühbuss, Heinrich, Dr.rer.nat., Hubert-Reissner-Strasse 5a, D-8032 Gräfelfing b. München (DE)**

EP 0 115 585 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist bekannt Schaum, insbesondere in wässerigen Systemen, mittels Antischaummitteln zu bekämpfen. In vielen industriellen Prozessen, in denen mit wässerigen Lösungen oder Suspensionen gearbeitet wird, ist das Schaumproblem eines der wesentlichsten Kriterien. Eine Vielzahl von Vorschlägen wurden in der Vergangenheit gemacht, solche Schäume zu vermeiden bzw. zu zerstören. Solche Vorschläge beschreiben den Einsatz von Silikonölen bzw. Silikonölemulsionen, Dispersionen von hydrophobem Siliciumdioxid, Compounds mit Fettsäureamiden, langkettigen Alkoholen, hydrophoben Polyglykolethern, Aluminium- und Magnesiumstearaten sowie Mischungen dieser Produkte untereinander.

Alle diese Vorschläge zielen auf eine Problemlösung in den unterschiedlichsten produktionstechnischen Bereichen, wie z.B. Abwasseraufbereitung, Papierherstellung, Zuckerrübenwäsche, Emulsionspolymerisationen sowie die Fertigung von Dispersionsfarben, wo der entstehende Schaum nicht nur die Produktion hemmt, sondern dem Anwender viele Probleme in bezug auf dekoratives Aussehen und schlechten Korrosionsschutz durch eingeschlossene Blasen mitbringt.

Die vorliegende Erfindung betrifft ein Verfahren zur Herstellung von Entschäumern für wässerige Medien, die als schaumdämpfend wirkende Verbindung Harnstoffe folgender Formel:

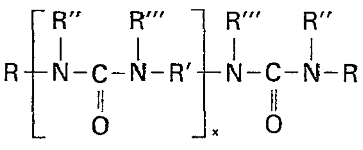

R = Alkyl $C_4$-$C_{30}$

R' = einfache chemische Bindung; Alkylen $C_2$-$C_{12}$ ein- bis zweikernige Arylreste, die am Arylrest zusätzliche Alkylgruppen $C_1$-$C_9$ aufweisen, Cycloalkylen

R'' = H, Alkyl $C_1$-$C_{24}$

R''' = H, $-CH_3-$

x = 0-5

enthalten, durch Umsetzen von mono- und/oder polyfunktionellen Isocyanaten mit mono- und/oder polyfunktionellen Aminen.

Die Herstellung von entschäumenden Kompositionen verlangt bisher ein hohes Mass an technischem Aufwand, hohen Energiekosten sowie eine sehr komplexe Gestaltung solcher Entschäumerformulierungen.

Aus der US-A-3965015 und der DE-A-2043088 sind Harnstoff- und Diharnstoffderivate als Weichmacher und schaumregulierende Mittel bekannt. Gemäss den US-A-3677963 und 3652453 werden die aus der US-A-3990905 bekannten Fettsäureamide durch Eingiessen ihrer heissen Schmelze in ein kaltes Trägermedium unter schnellem Rühren in Dispersionen übergeführt. Die in der DE-A-2558040 offenbarten Mittel zu Schaumbekämpfung bestehen aus Hydroxiamiden bzw. Hydroxi-Harnstoffen, deren Wirksamkeit aufgrund der hydrophilen OH-OH-Gruppen gering sein dürfte. Auch diese Verbindungen werden über ihren Schmelzpunkt hinaus erhitzt und danach durch Abkühlen in Dispersionen übergeführt. In allen Fällen erreicht indessen die Feinteiligkeit der Dispersionen nicht den wünschenswerten hohen Grad.

Nachteilig bei allen bisherigen Verfahren ist das rasche Absetzen der dispergierten Festkörper aus dem Trägermedium. Dies tritt besonders bei hydrophoben Kieselsäuren verstärkt auf. Ein weiterer Nachteil dieser Verfahren ist darin zu sehen, dass, bedingt durch hohe Tensidkonzentrationen im schäumenden Medium, eine Benetzung der hydrophoben Partikel erfolgt und damit ein Wirksamkeitsverlust bei Lagerung des mit Entschäumern versetzten Mediums eintritt. Dies ist besonders bei längerer Lagerung und erhöhter Temperatur zu bemerken.

Ziel der Entwicklung war es, einen möglichst universellen Entschäumer zu finden, welcher mit geringem technischen Aufwand ein hohes Mass an Sicherheit in bezug auf Entschäumung der unterschiedlichsten wässerigen Systeme bietet.

Diese Aufgabe ist bei einem Verfahren der oben beschriebenen Art erfindungsgemäss dadurch gelöst, dass die Reaktion der Isocyanate mit den Aminen in einem NCO/Amin-Äquivalentverhältnis von 0,5:1 bis 1:0,2 in situ in einem für die gebildeten Harnstoffe. Kein Lösungsmittel darstellendes organischen Trägermedium derart durchgeführt wird, dass die gebildeten Harnstoffe im organischen Trägermedium monidispers oder in mizellaren Strukturen anfallen, wobei die Umsetzungstemperatur unterhalb des Schmelzpunktes der resultierenden Harnstoffe gehalten wird.

Weitere vorteilhafte Weiterbildungen des Verfahrens der Erfindung sind in den abhängigen Ansprüchen 2 bis 6 angegeben.

Die Erfindung betrifft ferner Entschäumer, die nach den erfindungsgemässen Verfahren hergestellt sind, wie in den Ansprüchen 7 bis 12 dargestellt.

Überraschenderweise zeigte es sich, dass Harnstoffe, welche in situ in einem organischen Trägermedium bei verhältnismässig niedrigen Temperaturen hergestellt werden, ausgezeichnete entschäumende Eigenschaften für wässerige Medien aufweisen.

Harnstoffe mit entschäumenden Eigenschaften erhält man durch Zusammengeben, vorzugsweise äquivalenter Mengen, von Isocyanaten und Aminen in dem entsprechenden organischen Trägermedium bei Temperaturen unterhalb des Schmelzpunktes des Reaktionsproduktes.

Werden diese Harnstoffe jedoch über ihren Schmelzpunkt hinaus erhitzt oder bei Temperaturen oberhalb ihres Schmelzpunktes hergestellt, so besitzen sie noch eine unwesentliche entschäumende Wirkung.

Besonders bevorzugt sind Umsetzungsprodukte von Stearylisocyanat und/oder Palmitylisocyanat mit Oleylamin, Stearylamin und/oder Distearylamin. Des weiteren sind gut geeignet Umsetzungsprodukte von Gemischen aus Stearylamin mit Hexamethylendiamin und Toluylendiisocyanat. Im gleichen Sinne lassen sich die bekannten Polyamine, wie z.B. Ethylendiamin, Propandiamin, Butandiamin, Hexandiamin, 1,12-Dodecandiamin, 4,4'-Diaminodiphenylmethan, 3,3'-Dimethyl-4,4'-Diaminodiphenylmethan, p-Xylylendiamin, Isophorondiamin und Hydrazin sowie deren Gemische untereinander einsetzen.

Erfindungsgemäss sind ferner Umsetzungsprodukte von Monoaminen mit Polyisocyanaten, besonders mit den leichtzugänglichen Diisocyanaten, wie 2,4- und 2,6-Toluylendiisocyanat, Hexamethylendiisocyanat, Naphthylendiisocyanat und/oder 4,4'-Diaminodiphenylmethandiisocyanat. Als Monoisocyanate kommen alle, durch Phosgenierungsreaktion aus bekannten Monoaminen erhältlichen Isocyanate in Frage, besonders jedoch 2-Ethylhexylisocyanat, Isotridecylisocyanat, Palmityl-Stearylisocyanat sowie ein $C_{18}$-$C_{24}$-Alkylisocyanat.

Die als Entschäumer wirksamen Harnstoffe werden erfindungsgemäss in einem organischen Trägermedium hergestellt.

Die Konzentration an Harnstoff im Trägermedium ist, bedingt durch die resultierende Viskosität, limitiert und liegt zwischen 0,2 Gew.% und 50 Gew.%, vorzugsweise jedoch zwischen 1,0 Gew.% und 5,0 Gew.%, bezogen auf organisches Trägermedium.

Dieses organische Trägermedium kann in den meisten Fällen ein paraffinbasisches Spindelöl darstellen aber auch naphthenbasische und aromatische Mineralöle sowie Mischungen untereinander kommen in Frage. Von Fall zu Fall muss auch entschieden werden, ob Mischungen von mineralischen und nativen Ölen, wie z.B. Sojaöl oder Sonnenblumenöl, vorteilhaft sein können. Dies hängt in hohem Mass vom späteren Anwendungsgebiet des Entschäumers sowie von der Art des Harnstoffes ab. Des weiteren können niedrigsiedende Benzine, Xylol, Toluol, medizinische Weissöle, Ester von langkettigen Fettsäuren, wie Isobutylstearat und Isooctylstearat, Polyalkylenglykole und Kombinationen dieser Produkte mit Mineralöl verwandet werden.

Beim Einsatz der Entschäumer in Dispersionsfarben mit hohem Füllstoffanteil kann ein Zusatz von Polypropylenglykolen von Vorteil sein. Die erfindungsgemässen Entschäumer können zusätzlich Emulgatoren enthalten, die ihre Einarbeitung in wässerige System erleichtern oder die es ermöglichen, aus dem Entschäumer eine Stammemulsion herzustellen, die den zu entschäumenden Systemen zugesetzt wird. In diesem Falle ist der Entschäumer dadurch gekennzeichnet, dass zu den organischen Trägermedien 1-10 Gew.% nichtionogene Emulgatoren, welche einen HLB-Wert von 4 bis 18 aufweisen, zugesetzt sind.

Unter HLB-Wert versteht man eine Zahl, welche die Hydrophilie bzw. Hydrophobie eines Emulgators kennzeichnet. (W.C. Griffin „Classification of surface active agents by HLB", J. Soc. Cosmetic Chemists 1, 311 (1950)).

Es können Emulgatoren eines einzigen Types oder Emulgatorengemische verwendet werden.

Beispiele solcher Emulgatoren sind Fettalkoholethoxilate, Nonylphenolethoxilate, Sorbitanester, Fettsäureethoxilate.

Für Einsatzgebiete, in welchen organische Trägermedien störend sind, werden zweckmässigerweise Emulsionen erfindungsgemässer Entschäumer eingesetzt.

Diese Emulsionen werden nach bekannten Verfahren als Öl-in-Wasser oder Wasser-in-Öl Emulsionen formuliert.

Die nachfolgenden Beispiele 1-10 und 12-16 beschreiben die Herstellung erfindungsgemässer Entschäumer und geben die Herstellung einiger, dem Stand der Technik entsprechender Entschäumer an (Vergleichsbeispiele).

*Beispiel 1:*

282 g eines paraffinbasischen Mineralöls (Viskosität 12,2 $mm^2/s$ (66,5 SUS)/37,8° C) wurden in einem Rührgefäss auf ca. 40° C erhitzt und mit 1,14 g m-Xylylendiamin versetzt und so lange gerührt, bis das Amin vollständig gelöst war. Nach Zugabe von 4,86 g Octadecylisocyanat betrug die Nachreaktionszeit 30 Minuten.

*Beispiel 2:*

273 g eines technischen Weissöls (Viskosität 18,0 $mm^2/s$ (89,5 SUS)/37,8° C) wurden in einem Rührgefäss auf 35° C erhitzt und mit 1,82 g Octylamin versetzt und so lange gerührt, bis das Amin vollständig gelöst war. Nach Zugabe von 1,18 g 1,6-Hexamethylendiisocyanat betrug die Nachreaktionszeit 30 Minuten. Anschliessend wurden 3 g einer hydrophoben Kieselsäure (Ölzahl 180 g/100 g) eindispergiert.

*Beispiel 3:*

In einem Rühregefäss wurden 280,5 g eines naphthenbasischen Mineralöls (Viskosität 32,4 $mm^2/s$ (152 SUS)/37,8° C) vorgelegt, auf 50° C erhitzt und mit 1,26 g Oleylamin versetzt und so lange gerührt, bis das Amin vollständig gelöst war. Nach Zugabe von 1,74 g Octadecylisocyanat betrug die Nachreaktionszeit 30 Minuten bei 50° C. Anschliessend wurden 1,5 g Polydimethylsiloxan (Viskosität 200 $mm^2/s$ (200 cSt)/25° C) zugegeben, homogenisiert und auf 30° C abgekühlt.

*Beispiel 4:*

120 g eines naphthenbasischen Mineralöls (Viskosität 32,1 $mm^2/s$ (151 SUS)/37,8° C) wurden in einem Rührgefäss auf 50° C erhitzt, mit 3,33 g Octadecylamin versetzt und so lange gerührt, bis das Amin vollständig gelöst war. Nach Zugabe von 1,17 g Xylylendiisocyanat betrug die Nachreaktion 30 Minuten, wobei die Temperatur von 50° C auf 55° C stieg.

Anschliessend wurden nacheinander 160,5 g Polypropylenglykol (Molekulargewicht ca. 1000) und 15 g Sorbitanmonooleat zugegeben, homogenisiert und auf ca. 30° C abgekühlt.

*Beispiel 5:*

In einem Rührgefäss wurden 105 g eines paraffinbasischen Mineralöls (Viskosität 26,4 $mm^2/s$

(125 SUS)/37° C) mit 0,63 g Diethylentriamin bei 25° C versetzt und so lange gerührt, bis das Amin vollständig gelöst war. Nach Zugabe von 5,37 g Octadecylisocyanat betrug die Nachreaktionszeit 30 Minuten, wobei die Temperatur auf 32° C anstieg.

Anschliessend wurden nacheinander 174 g eines Testbenzins (Siedebereich 150-195° C), 9 g einer hydrophoben Kieselsäure (Ölzahl 200 g/100 g) und 9 g eines Nonylphenolethoxilats (Nonylphenol + 6 EO) zugegeben und homogenisiert.

*Beispiel 6:*

285 g Isododecan wurden in einem Rührgefäss auf 80° C erhitzt und mit 4,53 g Octadecylamin versetzt und so lange gerührt, bis das gesamte Amin gelöst war.

Nach Zugabe von 1,47 g 4-Methyl-m-phenylendiisocyanat betrug die Nachreaktionszeit 15 Minuten. Nach Zugabe von 9 g eines Nonylphenolethoxilats (Nonylphenol + 7 EO) wurde homogenisiert und auf ca. 30° C abgekühlt.

*Beispiel 7:*

In einem Rührgefäss wurden 285 g eines raffinierten Sonnenblumenöls bei 25° C mit 2,55 g Octadecylisocyanat und 2,16 g 1,6-Hexamethylendiisocyanat versetzt und so lange gerührt, bis die Isocyanate vollständig gelöst waren. Nach Zugabe von 1,29 g 1,3-Diaminopropan betrug die Nachreaktionszeit 15 Minuten. Anschliessend wurden 9 g Sorbitanmonooleat zugegeben und weitere 15 Minuten homogenisiert.

*Beispiel 8:*

150 g eines naphthenbasischen Mineralöls (Viskosität 45,0 $mm^2/s$ (210 SUS)/37,8° C) wurden in einem Rührgefäss auf 60° C erhitzt und mit 0,54 g Hexamethylendiamin versetzt und so lange gerührt, bis das gesamte Amin gelöst war. Nach Zugabe von 1,46 g Octadecylisocyanat betrug die Nachreaktionszeit 30 Minuten. Anschliessend wurden nacheinander 9 g eines Nonylphenolethoxilats (Nonylphenol + 7 EO), 9 g einer hydrophoben Kieselsäure (Ölzahl 180 g/100 g) und 0,6 g eines Polydiemthylsiloxans (Viskosität 100 (100 cSt)/25° C) zugegeben, homogenisiert und auf 30° C abgekühlt.

*Beispiel 9:*

In einem Rührgefäss wurden 956,25 g eines paraffinbasischen Mineralöls (Viskosität 45,0 $mm^2/s$ (210 SUS) 37,8° C) auf 60° C aufgeheizt und mit 1,8 g Hexamethylendiamin versetzt, bis zur vollständigen Auflösung des Amins gerührt und mit 8,2 g Octadecylisocyanat versetzt. Die Nachreaktion betrug 15 Minuten. Anschliessend wurden 20 g eines Polyoxiethylen-Sorbit-Hexaoleats (HLB=10,2) und 5 g Tallölfettsäure zugegeben und homogenisiert.

In einem zweiten Rührgefäss wurden 250 g entmineralisiertes Wasser vorgelegt und auf 60° C aufgeheizt, in welches unter starkem Rühren die Harnstoffsuspension langsam zugegeben wurde.

Die Endemulgierung erfolgte mittels Zahnkolloidmühle bei 60° C. Nach Abkühlen unter Rühren auf 30° C wurde die Emulsion mittels Vakuum entlüftet.

*Beispiel 10:*

197,5 g eines technischen Weissöls (Viskosität (18,0 $mm^2/s$) 89,5 SUS/37,8° C) wurden in einem Rührgefäss mit 1,9 g Xylylendiamin bei 25° C versetzt und bis zur vollständigen Auflösung des Amins gerührt.

Nach Zugabe von 8,1 g Octadecylisocyanat betrug die Nachreaktion 30 Minuten. Anschliessend wurden 15 g Sorbitanmonooleat zugegeben und unter starkem Rühren langsam 275 g entmineralisiertes Wasser zugegeben. Nach Zugabe von 2,5 g 37%iger wässeriger Formaldehydlösung erfolgte die Endemulgierung mittels einer Zahnkolloidmühle.

Die fertige Emulsion wurde mittels Vakuum unter Rühren entlüftet.

*Beispiel 11:* (Vergleichsbeispiel)

In einem Rührgefäss wurden 130 g eines technischen Weissöls (Viskosität 18,0 $mm^2/s$ (89,5 SUS)/37,8° C) auf 170° C erhitzt und mit 1,82 g Octylamin versetzt und so lange gerührt, bis das Amin vollständig gelöst war. Nach Zugabe von 1,18 g 1,6-Hexamethylendiisocyanat und einer Nachreaktionszeit von 30 Minuten wurde das Reaktionsgemisch in einem zweiten Rührgefäss unter intensiven Rühren und Kühlung in 143 g, auf 20° C abgekühltem, technischen Weissöl eingetropft.

Anschliessend wurden 3 g einer hydrophoben Kieselsäure (Ölzahl 180 g/100 g) und 5 g eines Nonylphenolethoxilats (Nonylphenol + 6 EO) eindispergiert.

Produkte mit annähernd gleichen Eigenschaften gemäss den Beispielen 1 bis 10 wurden erreicht durch die in den Beispielen 12-16 angegebenen Formulierungen.

*Beispiel 12:*

232,5 g eines paraffinbasischen Mineraöls (Viskosität 12,2 $mm^2/s$ (66,5 SUS)/37,8° C) wurden in einem Rührgefäss auf 30° C erhitzt, mit 0,55 g Diaminopropan versetzt und so lange gerührt, bis das Amin vollständig gelöst war. Nach Zugabe von 4,45 g Octadecylisocyanat betrug die Nachreaktionszeit 30 Minuten. Anschliessend wurden 12,5 g Sorbitanmonooleat zugegeben und weitere 15 Minuten gerührt. (Schmelzpunkt des Reaktionsproduktes aus Diaminopropan und Octadecylisocyanat = 105° C).

*Beispiel 13:*

In einem Rührgefäss wurden 232,5 g eines naphthenbasischen Mineralöls (Viskosität 32,4 $mm^2/s$ (152 SUS)/37,8° C) vorgelegt, auf 40° C erhitzt und mit 0,98 g 1,4-Diaminobutan versetzt und so lange gerührt, bis das Amin vollständig gelöst war. Nach Zugabe von 9,80 g Octadecylisocyanat betrug die Nachreaktionszeit 30 Minuten. Anschliessend wurden 7,5 g eines

Nonylphenolethoxilats (Nonylphenol + 6 EO) und 2,5 g einer hydrophoben Kieselsäure (Ölzahl 180 g/100 g) eindispergiert.

*Beispiel 14:*

236,3 g eines technischen Weissöls (Viskosität 18,0 $mm^2/s$ (89,5 SUS)/37,8° C) wurden in einem Rührgefäss auf 35° C erhitzt und mit 3,16 g 1,12-Diaminododecan versetzt und so lange gerührt, bis das Amin vollständig gelöst war. Nach Zugabe von 4,68 g Octadecylisocyanat betrug die Nachreaktionszeit 30 Minuten. Anschliessend wurden 7,5 g Sorbitanmonooleat zugegeben und weitere 15 Minuten gerührt.

*Beispiel 15:*

In einem Rührgefäss wurden 225 g eines naphthenbasischen Mineralöls (Viskosität 17,1 $mm^2/s$ (85 SUS)/37,8° C) mit 0,4 g Hydrazinhydrat versetzt und unter intensivem Rühren 4,6 g Octadecylisocyanat zugegeben. Die Nachreaktionszeit betrug 3 Stunden. Anschliessend wurden 12,5 g eines Nonylphenolethoxilats (Nonylphenol + 7 EO) und 7,5 g einer hydrophoben Kieselsäure (Ölzahl 180 g/100 g) eindispergiert.

*Beispiel 16:*

230 g Isododecan wurden in einem Rührgefäss auf 60° C erhitzt, mit 5,73 g Octadecylamin versetzt und so lange gerührt, bis das Amin vollständig gelöst war. Nach Zugabe von 1,78 g 1,6-Hexamethylendiisocyanat betrug die Nachreaktionszeit 45 Minuten. Nach Zugabe von 12,5 g Sorbitanmonooleat wurde homogenisiert und auf 30° C abgekühlt.

*Vergleichsprodukte*

Vergleichsprodukte A, B und C sind Entschäumerzubereitungen gemäss der US-PS 40 21 365 (Beispiel 1, 2 und 3).

Die Vergleichsprodukte D und E sind handelsübliche Entschäumer auf Mineralölbasis, welche nach folgenden Verfahren hergestellt wurden.

Vergleichsprodukt D

In einem Rührgefäss wurden 217,5 g eines naphthenbasischen Mineralöls (Viskosität 32,4 $mm^2/s$ (152 SUS)/38° C) vorgelegt und mittels Schnellrührer 25 g einer hydrophoben Kieselsäure und 7,5 g eines Emulgators (Nonylphenol + 7 EO) eindispergiert.

Vergleichsprodukt E

In einem beheizbaren Rührgefäss wurden 232,5 g eines naphthenbasischen Mineralöls (Viskosität 46,1 $mm^2/s$ (215 SUS)/38° C) vorgelegt und nacheinander 5 g einer hydrophoben Kieselsäure, 7,5 g Magnesiumstearat und 7,5 g eines Emulgators (Nonylphenol + 7 EO) unter Rühren eingearbeitet. Nach Aufheizen auf 100-105° C wird die Temperatur 1 h gehalten. Anschliessend wird unter Rühren auf ca. 40° C abgekühlt.

*Anwendungstechnische Prüfung*

(Angaben in Gewichtsteilen = T)

1. Dispersionsfarbe

| | |
|---|---|
| Vinylacetat/Vinylester-Copolymerisat Dispersion 50%ig | 121,5 T |
| Natriumpolyphosphat | 5,0 T |
| Ammoniumpolyacrylat | 1,5 T |
| Ammoniak | 1,5 T |
| Konservierungsmittel | 0,5 T |
| Testbenzin | 6,0 T |
| Dipropylenglykolmethylether | 2,0 T |
| Titandioxid | 61,0 T |
| Calciumcarbonat | 207,0 T |
| Talkum | 22,0 T |
| Verdickungsmittel | 2,5 T |
| Wasser | 68,0 T |
| Entschäumer | 1,0 T |

2. Glanzlack

| | |
|---|---|
| Acrylpolymer Dispersion (50%ig) | 265,5 T |
| Titandioxid | 125,0 T |
| Propylenglykol | 34,5 T |
| Ethylglykol | 9,2 T |
| Dipropylenglykolmethylether | 7,5 T |
| Hydroxyethylcellulose 2%ig in Wasser | 50,0 T |
| Natriumpolyacrylat | 4,0 T |
| Konservierungsmittel | 0,5 T |
| Wasser | 34,5 T |
| Entschäumer | 2,5 T |

3. Seidenglanzfarbe

| | |
|---|---|
| Styrol/Acrylatdispersion (50%ig) | 358,4 T |
| Titandioxid | 143,2 T |
| Calciumcarbonat | 143,2 T |
| Hydroxyethylcellulose 2%ig in Wasser | 70,0 T |
| Butylglykol | 21,6 T |
| Hochethoxilierter z.T. ungesättigter Fettalkohol | 18,0 T |
| Natriumpolyphosphat | 3,8 T |
| Ammoniumpolyacrylat | 0,8 T |
| Konservierungsmittel | 2,4 T |
| Testbenzin | 1,8 T |
| Ammoniak | 0,4 T |
| Entschäumer | 2,4 T |
| Wasser | 32,9 T |

Folgende anwendungstechnische Prüfungen wurden mit den vorstehend aufgeführten Dispersionsfarben durchgeführt:

a) Prüfung der fertigen Farbe

Gleich nach der Herstellung der Dispersionsfarbe bzw. nach einer Wärmelagerung von 7 Tagen bei 50° C werden 80 Teile der Farbe mit 20 Teilen Wasser gemischt und jeweils 1 Minute bei 2000 Upm mit einem Dissolver (Dispergierscheibe ⌀ 40 mm) gerührt.

Anschliessend wird das Gewicht von 50 ml dieses Gemisches bestimmt. Je höher das Gewicht der Probe, um so niedriger ist der Luftgehalt, um so besser also die Wirkung des Entschäumers.

b) Test mit der Schaumwalze

(auf Hart-PVC-Penetrationskontrastkarte 500 $cm^2$)

50 g der Dispersionsfarbe wird auf die Penetrationskontrastkarte gegeben und mit einer Schaumwalze so gleichmässig verteilt, dass 12,5 g

nasser Farbe (= 250 g/m²) verbleiben. Durch die Verwendung der aus offenporigen Polyurethanschaum bestehenden Schaumwalze (Breite 6 cm - Ø 7 cm), werden nicht nur die in der Farbe eingeschlossenen Schaumbläschen beurteilt, sondern zusätzlich die Luft, welche ähnlich der Pinselapplikation, in den Anstrich eingearbeitet wird. Nach dem Trocknen wird der Anstrich auf Lufteinschlüsse (Blasenbildung) visuell nach folgender Vergleichsskala beurteilt:

1 = kein Lufteinschluss
2 = sehr wenig Lufteinschluss
3 = wenig Lufteinschluss
4 = mässiger Lufteinschluss
5 = starker Lufteinschluss
6 = sehr starker Lufteinschluss

Die Ergebnisse sind in Tabelle I und II zusammengestellt und zeigen deutlich die Überlegenheit der erfindungsgemässen Mittel. Beispiel 11 zeigt den deutlichen Abfall der Wirksamkeit gegenüber dem gleichartig aufgebauten Beispiel 2, wenn der Harnstoff aus der Schmelze hergestellt wurde.

*Tabelle I*

*Entschäumerprufung (direkt nach Herstellung der Farbe)*

| | Dispersionsfarbe | | Glanzlack | | Seidenglanz | |
|---|---|---|---|---|---|---|
| | Lufteinschluss (Gew.%) | Applikation auf Kontrastkarte | Lufteinschluss (Gew.%) | Applikation auf Kontrastkarte | Lufteinschluss (Gew.%) | Applikation auf Kontrastkarte |
| Vergleichsprodukt A | 15,5 | 4 | 16,3 | 4 | 20,4 | 4 |
| Vergleichsprodukt B | 13,2 | 3 | 15,1 | 3 | 18,9 | 3 |
| Vergleichsprodukt C | 14,6 | 3 | 15,9 | 3 | 19,1 | 3 |
| Vergleichsprodukt D | 12,9 | 2-3 | 14,8 | 3 | 18,5 | 2-3 |
| Vergleichsprodukt E | 13,7 | 3-4 | 15,6 | 3-4 | 19,3 | 4 |
| Beispiel 1 | 13,5 | 3 | 14,6 | 3 | 19,5 | 3 |
| Beispiel 2 | 12,8 | 3-4 | 15,3 | 3-4 | 18,1 | 3 |
| Beispiel 3 | 14,1 | 3 | 15,9 | 3-4 | 19,8 | 3-4 |
| Beispiel 4 | 10,7 | 1-2 | 10,1 | 2 | 12,5 | 2 |
| Beispiel 5 | 9,9 | 1 | 9,6 | 1 | 9,8 | 1 |
| Beispiel 6 | 10,2 | 2 | 9,2 | 1 | 10,1 | 1 |
| Beispiel 7 | 11,1 | 2 | 10,5 | 2 | 9,4 | 1 |
| Beispiel 8 | 10,4 | 2 | 9,8 | 2 | 12,3 | 2 |
| Beispiel 9 | 12,5 | 2 | 11,1 | 2-3 | 13,5 | 2 |
| Beispiel 10 | 12,8 | 2-3 | 10,6 | 2-3 | 11,2 | 1-2 |
| Beispiel 11 | 25,3 | 5 | 22,1 | 5 | 25,7 | 5-6 |

*Tabelle II*

*Entschäumerprüfung (nach 7 Tagen Wärmelagerung bei 50°C)*

| | Dispersionsfarbe | | Glanzlack | | Seidenglanz | |
|---|---|---|---|---|---|---|
| | Lufteinschluss (Gew.%) | Applikation auf Kontrastkarte | Lufteinschluss (Gew.%) | Applikation auf Kontrastkarte | Lufteinschluss (Gew.%) | Applikation auf Kontrastkarte |
| Vergleichsprodukt A | 25,1 | 5 | 28,2 | 5 | 28,9 | 5 |
| Vergleichsprodukt B | 21,4 | 4-5 | 26,5 | 5 | 30,1 | 5 |
| Vergleichsprodukt C | 23,8 | 5 | 24,8 | 4-5 | 29,5 | 4-5 |
| Vergleichsprodukt D | 24,5 | 5 | 26,3 | 5 | 30,6 | 5 |
| Vergleichsprodukt E | 22,3 | 5 | 27,6 | 5-6 | 31,4 | 5-6 |
| Beispiel 1 | 18,6 | 3-4 | 22,7 | 3-4 | 27,1 | 3-4 |
| Beispiel 2 | 20,3 | 4 | 23,6 | 4 | 25,8 | 3-4 |
| Beispiel 3 | 20,1 | 3-4 | 23,1 | 4 | 28,3 | 4 |
| Beispiel 4 | 11,6 | 1-2 | 10,9 | 2 | 14,1 | 2-3 |
| Beispiel 5 | 10,3 | 1 | 10,3 | 1-2 | 12,0 | 1-2 |
| Beispiel 6 | 10,5 | 2 | 9,9 | 1 | 11,3 | 1 |
| Beispiel 7 | 11,9 | 2 | 10,8 | 2 | 10,6 | 1-2 |
| Beispiel 8 | 12,1 | 2-3 | 10,2 | 2-3 | 13,7 | 2-3 |
| Beispiel 9 | 12,8 | 2 | 12,8 | 3 | 15,2 | 2-3 |
| Beispiel 10 | 13,2 | 2-3 | 11,9 | 3 | 12,4 | 2 |
| Beispiel 11 | 27,4 | 6 | 29,5 | 5 | 30,8 | 5-6 |

*Anwendung der erfindungsgemässen Entschäumer bei der Emulsionspolymerisation*

Ein 500 ml Dreihalskolben mit Rührer, Tropftrichter, Rückflusskühler, Thermometer und Stickstoffleitung wird evakuiert und mit Stickstoff gefüllt. Dann gibt man 5 g Polyvinylalkohol zu und löst diesen in 100 ml destilliertem Wasser unter Rühren bei 60° C auf. Anschliessend fügt man noch 2,2 g ethoxiliertes Nonylphenol sowie 0,4 g Ammoniumperoxodisulfat und 0,46 g Natriumacetat zu.

In diese, auf 72° C aufgeheizte, Lösung lässt man 25 g frisch destilliertes Vinylacetat zutropfen und regelt dann das Wasserbad auf 80° C. Sobald die Innentemperatur 75° C erreicht hat, lässt man weitere 75 g Vinylacetat mit einer solchen Geschwindigkeit zutropfen, dass bei mässigem Rückfluss die Innentemperatur zwischen 79 und 83° C liegt. Anschliessend wird nochmals 0,1 g Ammoniumperoxodisulfat in 1 ml destilliertem Wasser zugegeben. Der Rückfluss lässt sehr bald nach, und die Innentemperatur steigt auf 86° C an. Man lässt dann noch 30 Minuten bei einer Wasserbadtemperatur von 80° C weiter polymerisieren. Zur Entfernung von Restmonomeren wurde die Dispersion mit 0,2 Gew.% eines Entschäumers gemäss Beispiel 7 versetzt und ein leichtes Vakuum angelegt.

Die mit Entschäumer versetzte Dispersion zeigte im Gegensatz zu einer Dispersion ohne Entschäumer kein Überschäumen bei der Monomerentfernung unter Vakuum.

## Patentansprüche

1. Verfahren zur Herstellung von Entschäumern für wässerige Medien, die als schaumdämpfend wirkende Verbindung Harnstoffe folgender Formel:

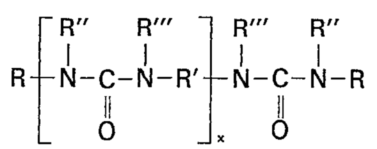

R = Alkyl $C_4$-$C_{30}$

R′ = einfache chemische Bindung; Alkylen $C_2$-$C_{12}$ ein- bis zweikernige Arylreste, die am Arylrest zusätzliche Alkylgruppen $C_1$-$C_9$ aufweisen, Cycloalkylen

R″ = H, Alkyl $C_1$-$C_{24}$

R‴ = H, $-CH_3-$

x = 0-5

enthalten, durch Umsetzen von mono- und/oder polyfunktionellen Isocyanaten mit mono- und/oder polyfunktionellen Aminen, dadurch gekennzeichnet, dass die Reaktion der Isocyanate mit den Aminen in einem NCO/Amin-Äquivalentverhältnis von 0,5:1 bis 1:0,2 in situ in einem für die gebildeten Harnstoffe kein Lösungsmittel darstellendes organischen Trägermedium derart durchgeführt wird, dass die gebildeten Harnstoffe im Trägermedium monodispers oder in mizellarer Struktur anfallen, wobei die Umsetzungstemperatur unterhalb des Schmelzpunktes der resultierenden Harnstoffe gehalten wird.

2. Verfahren nach Anspruch 1, dadurch gekennzeichnet, dass die Harnstoffe in Konzentrationen von 0,2-50 Gew.% im organischen Trägermedium hergestellt sind.

3. Verfahren nach Anspruch 1-2, dadurch gekennzeichnet, dass den Entschäumern 1-10 Gew.% nichtionogene Emulgatoren, welche einen HLB-Wert von 4 bis 18 aufweisen, zugesetzt sind.

4. Verfahren nach Anspruch 1-3, dadurch gekennzeichnet, dass die Harnstoffe enthaltenden Entschäumer in Wasser-in-Öl oder Öl-in-Wasser Emulsionen überführt werden.

5. Verfahren nach Anspruch 1-4, dadurch gekennzeichnet, dass als organische Trägermedium hydrophobe organische Verbindungen aus der Gruppe Brennöle, Mineraldichtungsöle, Paraffinöle, Naphthenöle, Cyclohexan, Xylol, Toluol und Dodecan ausgewählt sind.

6. Verfahren nach Anspruch 1-5, dadurch gekennzeichnet, dass dem organischen Trägermedium 1-30 Gew.% Polypropylenglykole mit Molekulargewichten von 800 bis 4000 zugesetzt sind.

7. Entschäumer, hergestellt gemäss dem Verfahren nach Anspruch 1-6, dadurch gekennzeichnet, dass er als schaumdämpfend wirkende Verbindung Harnstoff der Formel

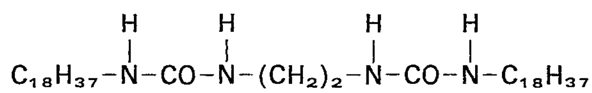

enthält.

8. Entschäumer, hergestellt gemäss dem Verfahren nach Anspruch 1-6, dadurch gekennzeichnet, dass er als schaumdämpfend wirkende Verbindung Harnstoff der Formel

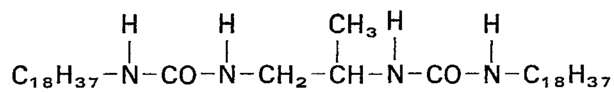

enthält.

9. Entschäumer, hergestellt gemäss dem Verfahren nach Anspruch 1-6, dadurch gekennzeichnet, dass er als schaumdämpfend wirkende Verbindung Harnstoff der Formel

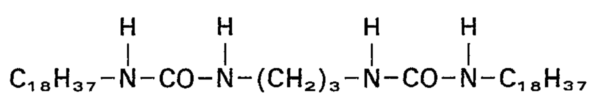

enthält.

10. Entschäumer, hergestellt gemäss dem Verfahren nach Anspruch 1-6, dadurch gekennzeichnet, dass er als schaumdämpfend wirkende Verbindung Harnstoff der Formel

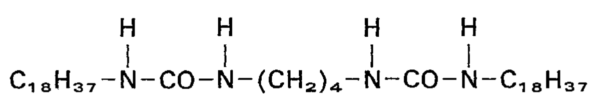

enthält.

11. Entschäumer, hergestellt gemäss dem Verfahren nach Anspruch 1-6, dadurch gekennzeichnet-

net, dass er als schaumdämpfend wirkende Verbindung Harnstoff der Formel

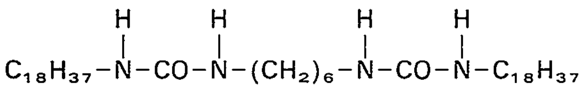

enthält.

12. Entschäumer, hergestellt gemäss dem Verfahren nach Anspruch 1-6, dadurch gekennzeichnet, dass er als schaumdämpfend wirkende Verbindung Harnstoff der Formel

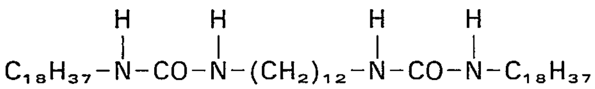

enthält.

## Revendications

1. Procédé de préparation d'antimousses pour des milieux aqueux, qui contiennent comme composés agissant contre la mousse des urées de formule suivante:

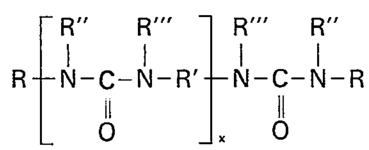

R = un groupe alcoyle en $C_4$ à $C_{30}$,
R' = une liaison chimique simple, un groupe alcoylène en $C_2$ à $C_{12}$, des groupes aryle à un ou deux noyaux qui portent en outre sur le groupe aryle des groupes alcoyle en $C_1$ à $C_9$, ou un groupe cycloalcoylène,
R'' = H ou un groupe alcoyle en $C_1$ à $C_{24}$,
R''' = H ou $-CH_3$,
x = de 0 à 5,

par réaction d'isocyanates mono- et/ou polyfonctionnels avec des amines mono- et/ou polyfonctionnelles, caractérisé en ce que la réaction des isocyanates avec les amines dans un rapport d'équivalents NCO/amine de 0,5:1 à 1:0,2 est effectuée in situ dans un milieu de support organique non solvant pour les urées formées, de telle sorte que les urées formées précipitent dans le milieu de support dans un état monodispersé ou dans une structure micellaire, la température de réaction étant maintenue au-dessous du point de fusion des urées résultantes.

2. Procédé selon la revendication 1, caractérisé en ce que les urées sont préparées en des concentrations de 0,2 à 50% en poids dans le milieu de support organique.

3. Procédé selon l'une des revendications 1 et 2, caractérisé en ce que de 1 à 10% d'émulsifiants non ionogènes, ayant une valeur HLB de 4 à 16, sont ajoutés aux antimousses.

4. Procédé selon l'une des revendications 1 à 3, caractérisé en ce que les antimousses contenant des urées sont transformés en des émulsions eau-dans-l'huile ou huile-dans-l'eau.

5. Procédé selon l'une des revendications 1 à 4, caractérisé en ce que comme milieux de support organiques, on choisit des composés organiques hydrophobes du groupe des huiles combustibles, des huiles minérales d'étanchéité, des huiles de paraffine, des huiles naphténiques, le cyclohexane, le xylène, le toluène et le dodécane.

6. Procédé selon l'une des revendications 1 à 5, caractérisé en ce qu'on ajoute au milieu de support organique de 1 à 30% en poids de polypropylèneglycols ayant une masse moléculaire de 800 à 4000.

7. Antimousse, préparé par le procédé selon l'une des revendications 1 à 6, caractérisé en ce que, comme composé agissant contre la mousse, il contient une urée de formule:

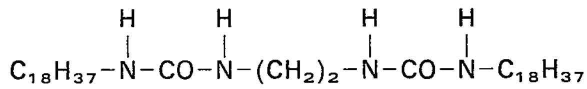

8. Antimousse, préparé par le procédé selon l'une des revendications 1 à 6, caractérisé en ce que, comme composé agissant contre la mousse, il contient une urée de formule:

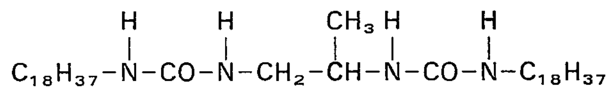

9. Antimousse, préparé par le procédé selon l'une des revendications 1 à 6, caractérisé en ce que, comme composé agissant contre la mousse, il contient une urée de formule:

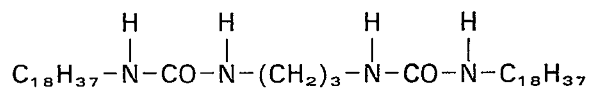

10. Antimousse, préparé par le procédé selon l'une des revendications 1 à 6, caractérisé en ce que, comme composé agissant contre la mousse, il contient une urée de formule:

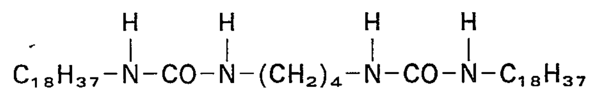

11. Antimousse, préparé par le procédé selon l'une des revendications 1 à 6, caractérisé en ce que, comme composé agissant contre la mousse, il contient une urée de formule:

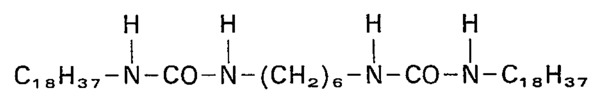

12. Antimousse, préparé par le procédé selon l'une des revendications 1 à 6, caractérisé en ce que, comme composé agissant contre la mousse, il contient une urée de formule:

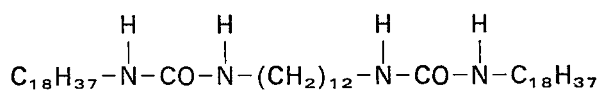

## Claims

1. Process for the production of de-foamers for aqueous media containing as activ de-foaming component ureas of the following formula

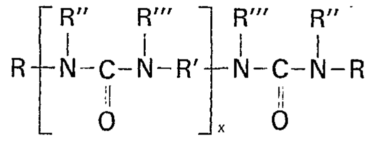

R = alkyl $C_4$-$C_{30}$;

R′ = single chemical bond $C_2$-$C_{12}$-alkylen uninuclear and/or dipyrenous aryl with substitution by $C_1$-$C_9$ alkyl groups cycloalkylene;

R″ = H, alkyl $C_1$-$C_{24}$;

R‴ = H, $-CH_3-$;

x = 0-5

by reacting mono- and/or polyfunctional isocyanates with mono- and/or polyfunctional amines, comprising reacting the isocyanates with the amines in a NCO/Amin ratio of 0,5:1 up to 1:0,2 in situ in an organic carrier media representing no true solvent for the resulting ureas at temperatures lying below the melting point of said resulting ureas, whereby the formed ureas are produced in the carrier media monodispersed or in micellar structure.

2. Process according to claim 1, wherein said urea is prepared in concentrations from 0.2-50% by weight in said organic carrier medium.

3. Process according to claim 1-2, comprising adding to the de-foamer from 1 to 10% by weight non-ionoganic emulsifier, displaying an HLB-value from 4 to 18.

4. Process according to claim 1-3, wherein an emulsifier containing said urea comprises water-in-oil or oil-in-water emulsions.

5. Process according to claim 1-4, wherein said organic carrier medium is selected from hydrophobic organic compounds from the group consisting of fuel oils, mineral sealing oils, paraffin oils, naphthene oils, cyclohexane, xylene, toluene, and dodecane.

6. Process according to claim 1-5, further comprising adding to said organic carrier medium from 1 to 30% by weight polypropyleneglycol with molecular weights from 800 to 5000.

7. De-foamer produced by the process according to claim 1-6, containing as active component urea of the formula

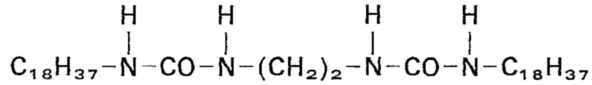

8. De-foamer produced by the process according to claim 1-6, containing as active de-foaming component urea of the formula

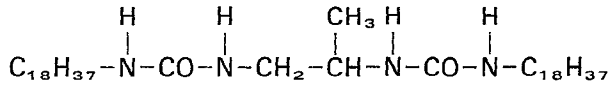

9. De-foamer produced by the process according to claim 1-6, containing active de-foaming component urea of the formula

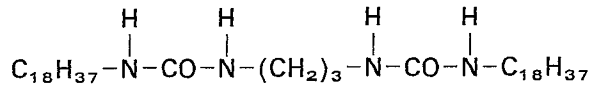

10. De-foamer produced by the process according to claim 1-6, containing as active de-foaming component urea of the formula

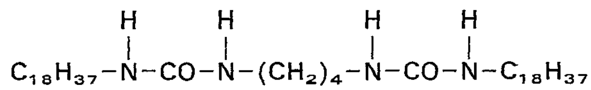

11. De-foamer produced by the process according to claim 1-6, containing as active de-foaming component urea of the formula

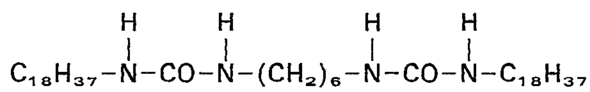

12. De-foamer produced by the process according to claim 1-6, containing as active de-foaming component urea of the formula